# EUROPEAN PATENT SPECIFICATION

(11) **EP 3 046 521 B1**
(45) Date of publication and mention of the grant of the patent: **10.11.2021**
(21) Application number: 14786138.9
(22) Date of filing: 19.09.2014
(51) Int. Cl.: A61F 13/02, A61F 13/00

(54) **DEVICE**
VORRICHTUNG
DISPOSITIF

(30) Priority: 20.09.2013 GB 201316769
(43) Date of publication of application: 27.07.2016
(73) Proprietor: Crawford Healthcare Ltd., Knutsford, Cheshire WA16 0BE (GB)
(72) Inventor: STEPHENSON, Christian, Wilmslow Cheshire SK9 2EP (GB)
(74) Representative: Simmons & Simmons
(86) International application number: PCT/EP2014/070031
(87) International publication number: WO 2015/040178

(56) References cited:
- EP-A1- 2 572 737
- US-A1- 2008 132 820
- US-A1- 2010 318 052
- US-A1- 2012 238 933

## Description

The present invention relates to a method of manufacturing a conformable absorbent wound dressing comprising a heat-plasticising process.

The term 'absorbent device' as used herein includes a reference to a medical absorbent device, such as a wound dressing for chronic and burn wounds, which comprises an absorbent component, as defined herein.

The term 'absorbent component' as used herein refers to any conformable component of an absorbent device, which is used for the absorption and retention in it of one or more bodily fluids from a body area, for example wound exudate from tissue in a wound. The absorbent component consists essentially of or comprises an absorbent material. The absorbent component is usually attached directly or indirectly to a fluid-impermeable backing layer of the absorbent device

The term 'absorbent material' as used herein refers to an absorbent material which is insoluble in, but absorbs a relevant bodily fluid and is essentially or is comprised in an absorbent component. Such a material may be in the form of a foam or sponge, fibres, an, often non-woven, fabric or a more random assembly of fibres, for example of randomly spun fibres, or in the form of a particulate.

The term "biocompatible" refers to any material which does not induce adverse effects such as immunological reactions and/or rejections in tissue in a human or animal.

The term "biodegradable" herein refers to any material which can be degraded and bioresorbed into, the physiological environment. Examples include absorbent materials, for example cellulose and cellulose-based materials, such as carboxymethylcellulose materials.

The term "container" herein in relation to any material in any component, for example an absorbent component, in an absorbent device, for example a wound dressing, refers to any conformable container which contains the material. It is often in the form of an optionally open-ended envelope, pouch or sachet, and may frequently have at least one, preferably one, liquid-permeable face, which typically comprises a layer of a spun-bonded or non-woven fabric, of a flexible and relatively inelastic polymer material.

The container may comprise a proximal (body-facing) cover of a layer or sheet material that is substantially insoluble in but permeable to aqueous bodily fluid, for example wound fluid, and lies between the contained material and the relevant body area, for example wound tissue in use. The container may also comprise a distal back layer or sheet material that is substantially insoluble in but permeable to aqueous bodily fluid, for example wound fluid, and lies distally of the contained material.

The term 'cushioning absorbent device' as used herein refers to a medical absorbent device, such as a wound dressing for chronic and burn wounds, which comprises an wound contact integer which also comprises a cushioning component, as defined herein.

The term 'cushioning component' as used herein refers to any conformable component of an absorbent device, which is used for relieving pressure on a body area, for example tissue in and around a wound, from which an absorbent component in the in the absorbent device absorbs a bodily fluid, for example wound exudate. The cushioning component consists essentially of, or comprises a cushioning material. The cushioning component is usually attached directly or indirectly to a fluid-impermeable backing layer of the absorbent device

The term 'cushioning material' as used herein refers to the cushioning material which is essentially or is comprised in a cushioning component. Such a material Is insoluble in a relevant bodily fluid, and a soft, flexible, resiliently elastic material, and may be in the form of a porous material, such as a foam, fibres, an, often non-woven, fabric or a more random assembly of fibres, for example of randomly spun fibres, or in the form of a particulate.

The term "distal" herein in relation to a face of a component in a device means that in use the face faces away from the body in general, or away from the body in general, or away from a wound in a wound dressing.

The term "fluidic contact" in relation to a first component and a second component in an absorbent device means that the first component is not directly in contact with the second component, but they are separated by a third component which is fluid-permeable, including moisture vapour transmitting. Examples include an absorbent material and a cushioning material of an absorbent device, where such a back layer of a stand-alone envelope between them holds them from direct contact.

The term "heat-plasticised" herein in relation to a proximal face of a component in a device of a flexible, conformable material means that the face has been subjected to heat, and optionally pressure, sufficient to soften it. Such a component in a device herein is in particular a wound contact layer, which normally forms the proximal face of the absorbent component or wound contact integer as defined herein, when a cushioning component is also present.

The term "indirectly" in relation to the attachment of a first component of an absorbent device to a second component means that the first component is attached directly to or comprised in a third component which is in turn attached directly to the second component.

The term "inner" herein in relation to any conformable container refers to any container which, together with its contents, is contained in another container, an "outer" container.

The term 'memory foam' as used herein with reference to the cushioning material which is essentially or is comprised in a cushioning component means a foam material, which is insoluble in a relevant bodily fluid, and is a soft, flexible, viscoelastic material with an open-cell solid structure. It is often a higher-density viscoelastic low-resilience hydrophilic polyurethane foam with additives which increase its viscosity and density, and matches pressure against it. In contact with a warm mammalian body, it softens in reaction to body heat, allowing it to mould to the body in a few minutes, yet slowly springs back to its original shape when the pressure is removed.

The term "non-biodegradable" herein refers to any material which cannot be degraded and bioresorbed into, the physiological environment.

Examples include polyalkylenes, such as polyethylene, polypropylene and polybutylene, and combinations and copolymers thereof.

The term "non-woven" herein in relation to fabrics in the absorbent component or the wound contact layer means that the majority of fibres in the fabric are neither woven nor knit together, and are typically manufactured by putting small fibres together to form a sheet or web, and then binding them together by mechanical interlocking, with an adhesive, or thermally, in particular by spin bonding.

The term "outer" herein in relation to any conformable container refers to any container which, together with any other contents, contains another container, an "inner" container.

The term "proximal" herein in relation to a face of a component in a device means that in use the face is a body-facing face in general, or a wound-facing face in a wound dressing.

By "substantially water-insoluble" in relation to any material herein is meant that the material is soluble in an aqueous medium to less than 1% w/w.

The term "wound" herein means any soft tissue with compromised integrity, including acute wounds, such as surgical wounds infectious disease wounds; chronic wounds, such as diabetic ulcers or venous leg ulcers; and burns.

The term "wound contact integer" as used herein means a liquid-permeable component in an absorbent device, which comprises an absorbent component and optionally a cushioning component. Such an integer is usually attached directly or indirectly to a liquid-impermeable backing layer.

The term "wound precursor" herein means any condition on or in the skin of a human or animal, or in the tissue underlying it, which in the absence of treatment is at substantial risk of developing into tissue of compromised integrity, such tissue including chronic wounds, including pressure sores, ulcers and infected wounds. Such conditions on or in the skin of a human or animal or in the tissue underlying it include discomfort, inflammation, pain and haematoma, which may be caused or exacerbated by pressure on the skin.

No statements made on belief herein, in particular as to the nature, physical form and properties of any integer or of any process shall be construed as limiting the present invention.

It is known to provide absorbent devices for example wound dressings, which are used to absorb one or more bodily fluids, for example wound exudate, from a body area, for example tissue around a wound, such as a leg or foot ulcer, or a pressure ulcer.

It is known to provide absorbent and cushioning devices for example cushioning wound dressings, which are used to absorb one or more bodily fluids, for example wound exudate, and to relieve pressure on a body area, for example tissue around a wound, such as a leg or foot ulcer, or a pressure ulcer.

Some tissue of compromised integrity, including pressure-sensitive chronic wounds, such as diabetic ulcers, venous leg ulcers, pressure sores, burn wounds and infected wounds on or in the skin of a human or animal or in the tissue underlying it, may be heavily exuding wounds.

In known absorbent devices, for example wound dressings, and known absorbent and cushioning devices for example cushioning wound dressings, the wound contact layer is liquid-permeable, and typically comprises at least one, preferably one, perforated layer or sheet, or layer of spun-bonded or non-woven fabric, of a flexible and relatively inelastic polymer material allows fluidic contact between the absorbent component and/or the absorbent material and the wound.

In some known absorbent devices and known absorbent and cushioning devices, there is a tendency for there to be friction between the wound contact layer and the body area, for example tissue in and/or around a wound, and in some cases, the wound contact layer may adhere to, the relevant body area, for example to wound and/or surrounding skin tissue on removal of the absorbent device or absorbent and cushioning device.

US 2008/132820 A1 concerns devices and methods for achieving hemostasis in patients who have received skin-penetrating wounds to the periphery, including the head, arms, and legs. US 2012/238933 A1 concerns a compression kit including an inner layer, comprising a foam material, and an outer layer, comprising a separate elastic compression bandage. US 2010/318052 A1 concerns a medical dressing comprising a backing layer and a self-supporting substrate. EP 2 572 737 A1 concerns a wound dressing for use in the treatment of skin wounds with some level of exudation.

There is thus a need for a device with an absorbent component which consists essentially of or comprises an absorbent material, in which a high absorbency and rate of absorption and retention of bodily fluids from a body area, for example wound exudate from tissue in a wound, can be achieved.

There is thus also a need for a device with an absorbent component and/or an absorbent material, and a wound contact layer, in which friction between the wound contact layer and the body area, for example tissue in and/or around a wound is reduced and/or the wound contact layer does not adhere to the relevant body area, for example to wound and/or surrounding skin tissue, on removal of 10 the absorbent device or absorbent and cushioning device.

Accordingly, it is an object of the present invention to provide a device which solves these problems.

We have surprisingly found that all the above disadvantages may be overcome by subjecting the proximal face of a wound contact layer in a device, which is often of a flexible, resiliently elastic material, to heat, and pressure, sufficient to soften it.

This improves the absorbency and rate of absorption and retention of bodily fluids from a body area, for example wound exudate, and reduces friction between the wound contact layer and the body area, for example tissue in and/or around a wound, and reduces adherence to the peri-wound skin or wound. Effectively the proximal face of the wound contact layer in a device becomes very slippery.

Accordingly, the present invention provides a method of manufacturing a conformable absorbent wound dressing according to claim 1. As noted above, "heat-plasticised" herein in relation to a proximal face of a component in a device of a flexible, resiliently elastic material means that the face has been subject to heat, and pressure, sufficient to soften it.

The conformable absorbent device may be used in the treatment of chronic wounds, such as venous leg ulcers, pressure-sensitive chronic wounds, such as pressure sores and diabetic foot ulcers, or precursors of any such wounds. The conformable absorbent device has improved absorbency and rate of absorption and retention of bodily fluids from a body area, for example wound exudate, and reduced friction between the wound contact layer and the body area, for example tissue in and/or around a wound, and reduced adherence to the peri-wound skin or wound compared with known absorbent devices and known absorbent and cushioning devices.

In the device, a wound contact layer normally fully overlies the proximal face of the absorbent component or wound contact integer as defined herein, when a cushioning component is also present. The backing layer will generally extend beyond the periphery of the distal face of the wound contact integer. The wound contact layer at least partially overlies the proximal face of the backing layer. Whilst the two components may be essentially coterminous, the backing layer will generally extend beyond the periphery of the wound contact layer.

Where it is desired to relieve pressure on the body area, for example tissue around a wound, such as a leg or foot ulcer, or a pressure ulcer, the device comprises a cushioning component, which consists essentially of, or comprises a cushioning material.

The cushioning component is often attached directly or indirectly to the proximal face of the fluid-impermeable backing layer of the device.

The cushioning component lies between the backing layer of the device and the absorbent component, which is intended to absorb a bodily fluid. The latter may be attached directly or indirectly to the cushioning component, and together with the cushioning component essentially is or is comprised in a wound contact integer.

The wound contact layer may be attached directly to the proximal face of the backing layer, for example, around its periphery by at least one layer of an adhesive material, often a pressure sensitive adhesive, for example a pressure sensitive acrylic adhesive, on the proximal face of the backing layer. This is generally the same adhesive on its proximal face that is used for the purpose of securing it to the absorbent component or the wound contact integer when an optional cushioning component is present.

It will be seen that the wound contact layer and the backing layer together sandwich the absorbent component or the wound contact integer when an optional cushioning component is present, and form a container, which is not a stand-alone envelope. It may optionally contain at least one inner container, as described hereinafter.

The backing layer often extends beyond the periphery of the wound contact layer. Where the at least one layer of adhesive is a pressure sensitive adhesive, for example a pressure sensitive acrylic adhesive, on its proximal face beyond the periphery of the wound contact layer it may be used for the purpose of securing it to the skin of a patient,

Alternatively the two integers may be mutually attached by thermal welding or lamination. Again, the backing layer often extends beyond the periphery of the wound contact layer, and it may bear on its proximal face a pressure sensitive adhesive, for example a pressure sensitive acrylic adhesive, on its proximal face beyond the periphery of the wound contact layer it may be used for the purpose of securing it to the skin of a patient,

Alternatively, in a preferred form of the device, the wound contact layer may form a fluid-permeable proximal cover layer in a container in the form of a stand-alone envelope, attached to a distal back layer. This may also contain the absorbent component as a conformable stand-alone inner container containing the absorbent material. The proximal cover layer and distal back layer are mutually attached, often around their co- extensive peripheries to form the stand-alone envelope, for example by at least one layer of an adhesive material, often a pressure sensitive adhesive, for example a pressure sensitive acrylic adhesive or by thermal welding or lamination. The wound contact and backing layers are not bonded to each other directly, but the distal back layer of the stand-alone envelope and the backing layer are bonded to each other directly, again by at least one layer of adhesive is a pressure sensitive adhesive, for example a pressure sensitive acrylic adhesive, or by thermal welding or lamination around the periphery of or across the back layer.

Again, the backing layer often extends beyond the periphery of the wound contact layer, and it may bear on its proximal face a pressure sensitive adhesive, for example a pressure sensitive acrylic adhesive, on its proximal face beyond the periphery of the back layer, to be used for the purpose of securing it to the skin of a patient,

Any part of a layer of pressure-sensitive adhesive which is used for the purpose of securing the device onto a body area will preferably have release papers, such as release papers release-coated with polymers such as silicone, acrylate or polyurethane. These cover the layer of pressure-sensitive adhesive when it is not in use.

The wound contact layer in a device is liquid-permeable, and typically comprises at least one, preferably one, perforated layer or sheet, or layer of spun-bonded or non-woven fabric, of a flexible and conformable polymer material, which is heat-plasticised. That is, at least the proximal face of the material has been subjected to heat, and optionally pressure, sufficient to soften it, for example in the heat-plasticising process comprised in the method of manufacturing an absorbent device of the present invention.

Where the layer is a layer of spun-bonded or non-woven fabric, it will often have a monofilament linear density of 0.1 to 30, preferably about 0.5 to 20, and more preferably 0.9 to 4, for example 1 3 to 5 decitex. It will have a strength of 0.8 to 2.2, such as 1 to 2, for example 1 .2 to 1 .8 cN/dtex.

The material of the wound contact layer, whether as a perforated layer or sheet, or layer of spun-bonded or non-woven fabric, is typically a flexible and relatively inelastic material. Examples of suitable materials include substantially water- insoluble, heat-plasticised, synthetic thermoplastic polymeric materials, such as such polyalkylenes, for example polyethylenes, polypropylenes and polybutylenes, polyoxalates, polyamides, silicones, polyurethanes, polyesters and copolymers and blends thereof.

The material of the wound contact layer, in whatever form, may conveniently be heat-plasticised in the heat-plasticising process comprised in the method of manufacturing an absorbent device of the present invention.

The conformable device is typically provided with a backing layer which is preferably a gas-permeable barrier layer which is capable of forming a relatively liquid-tight seal on the body, but preferably has a high moisture vapour transmission (MVT). The backing layer is impermeable to bodily fluid, for example wound exudate, for example by being a continuous layer of a substantially insoluble polymeric material which is impermeable to the relevant body fluid.

The material of the backing layer is typically a flexible and conformable material, which may be in the form of a polymer sheet. Examples of suitable materials include substantially water-insoluble synthetic thermoplastic polymeric materials, such as polyalkylenes, for example polyethylenes, polypropylenes and polybutylenes, polyoxalates, polyamides, silicones, polyurethanes, polyesters and copolymers and blends thereof.

The absorbent device, its absorbent component, and the absorbent material, which is essentially or is comprised in the absorbent component, and embodiments of the device which relate to the absorbent material and the absorbent component are now described in detail.

The structure, form and physical properties of the absorbent component, absorbent material and containers, including inner and outer containers, within the absorbent device have a significant effect on the absorbent performance of the absorbent device.

In all embodiments of an absorbent device, such as a wound dressing:
a) the absorbent component may consist essentially of an absorbent material, or b) the absorbent component may comprise the absorbent material, contained within a first container, which is a stand-alone envelope, optionally as an inner container contained in at least one outer second conformable container, the first conformable container and any second conformable container each having a proximal cover layer, which is permeable to bodily fluid, and each material is preferably present in the device as a layer.

Thus in the case of the device described immediately above in detail, the absorbent material may optionally be contained within a first conformable container, which is a stand-alone envelope, as an inner container contained in an outer second container formed by the wound contact layer and the backing layer together sandwiching the absorbent component, which is not a stand-alone envelope. Each has a proximal cover layer which is permeable to bodily fluid, and the absorbent material is preferably present in the device as a layer.

It is also desirable that the dimensional stability of any absorbent material is retained in use, as exudate is absorbed by it. This may be aided by enclosing the absorbent material in a conformable container.

Examples of suitable materials for an absorbent include
hydrophilic natural materials, for example cotton, cellulose, chitosan and wool; materials made from naturally occurring materials, for example alginate and cellulose-based materials, such as carboxymethylcellulose materials; and synthetic materials, such as polyurethane foam or sponge, hydrocolloids and hydrogels, and copolymers and blends thereof. Examples of suitable materials for an absorbent material also include specific synthetic polymers, such as pharmaceutically acceptable salts of poly(acrylic and/or methacrylic) acids, favourably cross-linked polyacrylate and polymethacrylate salts, for example alkali metal salts thereof, and copolymers and blends thereof. Preferred absorbent materials include cross-linked polyacrylate and polymethacrylate sodium salts, in particular cross-linked sodium polyacrylates, and blends thereof.

Typically the absorbency of these absorbent materials is of the order of 15 to 60, for example about 20 to 30 g/g of 0.9 % saline. Although this enables large quantities of bodily fluid (g/g of absorbent material in the device) to be absorbed into the absorbent material in the device, the nature and properties of this material prevent transfer of the fluid from the distal face of the absorbent component to any other potentially absorbent component, such as an optional cushioning component.

In a number of embodiments of the absorbent device, such as a wound dressing, the device comprises a wound contact integer, which comprises an absorbent component and an optional cushioning component, as defined, and
a) the cushioning component may often consist essentially of an elastically resilient cushioning material, or
b) the cushioning component may comprise the cushioning material, contained within a first container.

The structure, form and physical properties of the cushioning component, cushioning material and containers, including inner and outer containers, within the absorbent device depend significantly on whether the cushioning component is intended to absorb a bodily fluid from the absorbent component in use. In general, such absorption and retention in the cushioning component from the absorbent component in use may have a negative effect on the cushioning performance of the absorbent device.

It the effect is not significant, then the proximal face of the cushioning component may be in direct contact with the distal face of the absorbent component in use or in fluidic contact when in an optional cushioning component which comprises the cushioning material, contained within a first container, through the proximal cover layer of the container, which is permeable to bodily fluid.

The absorbent component in the device may comprise a specific absorbent material which is a specific synthetic polymer, such as the pharmaceutically acceptable salts of poly(acrylic and/or methacrylic) acids described above in detail. The nature and properties of this material prevent transfer of the fluid from the distal face of the absorbent component to any other potentially absorbent component, such as an optional cushioning component, which could absorb bodily fluid from the absorbent component in use. There is thus no significant negative effect on the cushioning performance of the absorbent device.

Thus, the proximal face of the cushioning component may be in direct contact with the distal face of the absorbent component in use or in fluidic contact when in an optional cushioning component which comprises the cushioning material, contained within a first container, through the proximal cover layer of the container, which is permeable to bodily fluid. In other cases, the absorbent component in the device does not comprise a specific absorbent material which is the above specific synthetic polymer, and the nature and properties of this material do not prevent transfer of fluid from the distal face of the absorbent component in use to the proximal face of an optional cushioning component which consist essentially of or comprises the cushioning material, for example contained within a first container.

Absorption and retention in the cushioning component from the absorbent component in use has a negative effect on the cushioning performance of the absorbent device.

The proximal face of the cushioning material should not be in direct or fluidic contact with the distal face of the absorbent material in the device, and it is necessary to prevent transfer of the fluid from the distal face of the absorbent material to the potentially absorbent cushioning material.

Either or both of the cushioning material and the absorbent material in the device should be contained within a container, preferably a stand-alone envelope, with a) in the case of the cushioning material, the proximal cover layer of the container, and optionally the back layer, being impermeable to bodily fluid, and
b) in the case of the absorbent material, the proximal cover layer of the container being permeable to bodily fluid, and optionally the back layer being impermeable to bodily fluid.

The absorbent component or the wound contact integer, which comprises an absorbent component and an optional cushioning component, as defined, is often attached directly to the proximal face of the backing layer of the device, by the layer of a pressure sensitive adhesive on the proximal face of the device backing layer, described hereinbefore.

Depending on the structure, form and physical properties of the components, material and containers, including inner and outer containers as described above, the proximal face of the backing layer of the device may be attached directly to the absorbent material, the cushioning material, or the back layer of a container containing either, as appropriate.

The absorbent material in the absorbent device may be in the form of a foam or sponge, fibres, an, often non-woven, fabric or a more random assembly of fibres, for example of randomly spun fibres, or preferably in the form of a particulate.

Where the absorbent material in an absorbent device is a particulate or in any other form that is not attached directly to a device backing layer, it is then contained in a conformable container.

Where the material (or any other component of the device) in use might adhere to, the relevant body area, for example to wound tissue on removal of the absorbent device, it is contained in such a container.

It may be preferred that the absorbent material in the absorbent device, irrespective of its form, is present in the device as a layer in a conformable container. The layer may typically have a mean pre-absorption thickness of the order of 0.3 to 10, for example 0.7 to 5 mm.

The container may be formed by a proximal (body-facing) fluid-permeable cover layer attached directly or indirectly to the backing layer. Preferably, it will be a stand-alone envelope, with a fluid-permeable cover layer and a back layer which may be liquid-permeable or liquid-impermeable, depending on the structure, form and physical properties of the components, materials and containers, including inner and outer containers as described above. As noted above, the proximal face of the wound contact layer which comprises a heat-plasticised flexible, conformable material, may be the fluid-permeable cover layer of a conformable container, preferably a stand-alone envelope. The proximal face of the container may have been subjected to heat, and optionally pressure, sufficient to soften it, for example in the heat-plasticising process comprised in the method of manufacturing an absorbent device of the present invention.

Such a wound contact layer, which forms the proximal face of the wound contact integer may be bonded to a conformable backing layer, to form the container, but is preferably bonded to an optionally fluid-permeable back layer, to form a standalone envelope, the distal face of which is typically is bonded to the conformable backing layer, for example with a pressure sensitive adhesive, as is described hereinabove.

Preferably there is provided a conformable absorbent device that comprises a particulate form of the substantially water-insoluble absorbent material. It will be appreciated that the absorbent material which is in the form of a particulate needs to be contained in a conformable container, preferably a standalone envelope. The stand-alone envelope is typically bonded to a conformable backing layer, for example with a pressure sensitive adhesive, as described hereinabove in relation to the absorbent material in general.

Suitable and preferred absorbent materials used in the absorbent component of the absorbent device are as so described hereinabove in relation to the absorbent material in general. The absorbent material is preferably present as a layer. Such a layer in a conformable container may typically have a mean pre-absorption thickness of the order of 0.3 to 10, for example 0.7 to 5 mm. The material may typically have a mean pre-absorption particle size of the order of 5 to 1000, for example 15 to 900 microns.

Where the absorbent material is a specific synthetic polymer, such as the pharmaceutically acceptable salts of poly(acrylic and/or methacrylic) acids described above in detail, the nature and properties of this material prevent transfer of the fluid from the distal face of the absorbent component, and this appears to be independent of the thickness of this layer.

There is also provided a conformable absorbent device that comprises a, for example non- woven, fabric of fibres of one or more, preferably one, substantially water-insoluble, often hydrophilic absorbent materials.

The absorbent material in the form of a, for example non-woven, fabric in the device may have a pre-absorption density of 2 to 8, and preferably 3 to 5, pounds/cubic foot (pcf). Suitable and preferred absorbent materials used in the absorbent component of the absorbent device are as so described hereinabove in relation to the absorbent material in general.

The absorbent material may be in the form of at least one, preferably one, sheet, optionally bonded to each other. The total layer of a sheet, or optionally bonded sheets, may typically have a mean pre-absorption thickness of the order of 0.3 to 10, for example 0.7 to 5 mm.

Where the absorbent material is a specific synthetic polymer, such as the pharmaceutically acceptable salts of poly(acrylic and/or methacrylic) acids described above in detail, the nature and properties of this material prevent transfer of the fluid from the distal face of the absorbent component, and again this appears to be independent of the thickness of this layer. Preferably, however, the sheet, or optionally bonded sheets, are contained in a conformable stand-alone envelope, which typically is bonded to the conformable backing layer, for example with a pressure sensitive adhesive, as is described hereinabove in relation to the absorbent material in general. There is also provided a conformable absorbent device that comprises a substantially water- insoluble, often hydrophilic absorbent material as a random assembly of fibres.

The absorbent material may be in the form of at least one, preferably one, piece of wadding, such as a cushion or pad.

The pre-absorption fibres may have a monofilament linear density of 0.1 to 30, preferably about 0.5 to 20, and more preferably 0.9 to 4, for example 1 3 to 5 decitex, and a strength of 0.8 to 2.2, such as 1 to 2, for example 1 .2 to 1 .8 cN/dtex. It is preferred that the absorbent material in the form of a random assembly of fibres.

Suitable and preferred absorbent materials used in the absorbent component of the absorbent device are as so described hereinabove in relation to the absorbent material in general. The absorbent material is preferably one or more spun materials, such as a spun- bonded hydrophilic polymeric material,

It is preferably a layer which may typically have a mean pre-absorption thickness of the order of 0.3 to 10, for example 0.7 to 5 mm. Where the absorbent material is a specific synthetic polymer, such as the pharmaceutically acceptable salts of poly(acrylic and/or methacrylic) acids described above in detail, the nature and properties of this material prevent transfer of the fluid from the distal face of the absorbent component, and this again appears to be independent of the thickness of this layer.

The at least one piece of wadding may be formed by spinning, optionally by electrospinning, or by melt-blowing. Spin-bonding of the component fibres is preferred. The random assembly of fibres may be attached or spun, in particular with spin-bonding of the fibres, directly onto the proximal surface of the backing layer or any cushioning component. The latter may have at least one discontinuous area of an adhesive for the purpose of securing the random assembly of fibres. The absorbent component and any cushioning component may be attached to the conformable backing layer, for example with a pressure sensitive adhesive Preferably, however, the random assembly of fibres is contained in a conformable stand-alone envelope, which is optionally an inner container contained within an outer stand-alone envelope, also with a fluid-permeable cover layer and a fluid- permeable back layer, which typically is bonded to the conformable backing layer, for example with a pressure sensitive adhesive, as is described hereinabove in relation to the absorbent material in general.

There is also provided an absorbent device that comprises a substantially water-insoluble, often hydrophilic absorbent material as a foam or sponge.

It is preferred that the absorbent material is in the form of an open-cell foam or sponge. The absorbent material may be in the form of at least one, preferably one, piece of open-cell foam or sponge, optionally bonded to each other.

Suitable and preferred absorbent materials used in the absorbent component of this absorbent device are as so described hereinabove in relation to the absorbent material in general. The absorbent material is preferably a layer, which may typically have a mean pre-absorption thickness of the order of 0.3 to 10, for example 0.7 to 5 mm. Where the absorbent material is a specific synthetic polymer, such as the pharmaceutically acceptable salts of poly(acrylic and/or methacrylic) acids described above in detail, the nature and properties of this material prevent transfer of the fluid from the distal face of the absorbent component, and again this appears to be independent of the thickness of this layer.

The absorbent component or any cushioning component may be attached to the conformable backing layer, for example with a pressure sensitive adhesive. Preferably, however, the piece of foam, or optionally bonded pieces, is contained in a conformable stand-alone envelope, which is optionally an inner container contained within an outer stand-alone envelope, also with a fluid-permeable cover layer and a fluid-permeable back layer, which typically is bonded to the conformable backing layer, for example with a pressure sensitive adhesive, as is described hereinabove in relation to the absorbent material in general.

It will be seen from the foregoing that the absorbent material may be used in the present absorbent component in a wide variety of forms

The function of any cushioning component in a medical device is typically to provide relief of pressure on pressure-sensitive areas of the body that are particularly subject to pressure. The absorbent component simultaneously absorbs exudate at a relatively high degree and rate of absorption and retention from a chronic wound, for example a pressure sore, an ulcer, such as a diabetic foot ulcer or venous leg ulcer, or a burn wound. Another function of the cushioning component in a medical device is typically to provide prophylaxis or treatment of wound precursors, as defined herein, to prevent pressure sores forming. These pressure-sensitive chronic wounds, such as diabetic ulcers or venous leg ulcers, or precursors of such wounds potentially occur in patients, for example with arthritis, diabetes, cardiovascular conditions and/or paralysis.

The corresponding cushioning absorbent device thus comprises a wound contact integer which also comprises a cushioning component distally of the absorbent component and usually attached directly or indirectly to a liquid-impermeable backing layer of the device.

Neither of the wound contact integer or the cushioning component is shaped to conform in use to the relevant part of the body. The wound contact integer is thus conveniently in a versatile lamellar shape such as a quadrilateral, such as a rectangle or oblong, or an equilateral, such as a square. Suitable cushioning components include conventional cushioning components having an architecture, for example a foam, and being of materials that make them elastically resilient. Such materials tend to be substantially water-insoluble, elastically resilient synthetic polymeric materials including, but not limited to, hydrophilic polyurethanes and silicones, and copolymers and blends thereof. Where the material of the cushioning component comprises a copolymer, examples of suitable copolymers include block copolymers of butylene with styrene and comonomers.

The cushioning component may comprise a cushioning material which is contained in its own conformable container, but typically consist essentially of an optional cushioning material. The structure, form and physical properties of any containers, including inner and outer containers that are appropriate to different absorbent materials and material of the cushioning component in the absorbent device, are described hereinabove in relation to these materials in general. If used with an absorbent material which is a specific synthetic polymer, such as the pharmaceutically acceptable salts of poly(acrylic and/or methacrylic) acids described above in detail, and the nature and properties of which prevent transfer of the fluid from the distal face of the absorbent component, even when the components are in fluidic contact, the cushioning materials suffer no or negligible loss of the initial elastic resilience and also demonstrate good dimensional stability in the present absorbent device.

In a fifth, preferred embodiment, a cushioning component consists essentially of a foam cushioning material, preferably one layer of a foam, in particular an open- cell foam.

The total layer may typically have a mean pre-absorption thickness of the order of less than 10 mm, for example 4 to 8, such as about 6 millimetres thick.

The mean pre-absorption pore density may be for example from about 2 to 8, particularly from about 3 to 5 pcf. Suitable and preferred materials used in the absorbent component and any cushioning component of this second embodiment of the absorbent device are as so described hereinabove in relation to the cushioning material in general.

In a sixth, less preferred embodiment, the absorbent component and any cushioning component consists of at least one, preferably one, piece of wadding in the form of a random assembly of fibres of an elastically resilient polymeric material, preferably connected by being spun-bonded together.

The total layer may typically have a mean pre-absorption thickness of the order of less than 20 mm, for example 3 to 6 mm. Such flexible fibres typically have a monofilament linear density of 0.1 to 30, preferably about 0.5 to 20, and more preferably 0.9 to 4, for example 1 3 to 5 decitex, and a strength of 0.8 to 2.2, such as 1 to 2, for example 1 .2 to 1 .8 cN/dtex.

Suitable materials include optionally spun-bonded synthetic materials, such as non-biodegradable polymers such as spun-bonded polyalkylenes, for example polyethylenes, polypropylenes and polybutylenes, polyoxalates, polyamides, polycarbonates, polystyrene, and copolymers and blends thereof,

The device optionally comprises a component, as defined herein, comprises a therapeutic agent.

For example, some materials used in the device, normally in the absorbent component, of the devices are inherently antimicrobial, including antibiotic and/or antimycotic, for example natural materials, such as chitosans and semi-synthetic materials, such as carboxymethylchitosans.

Inherently antimicrobial materials in the adhesive devices, for example carboxymethylchitosan and chitosan, in particular as a particulate, offer convenience in manufacturing the device, since a further therapeutic agent does not have to be incorporated into the device.

Some materials used in the device, normally in the absorbent component, of the devices are inherently haemostatic, for example natural materials, such as gelatine, in particular as an open-cell foam or sponge, collagens, fibrin sealants and chitosans; and semi-synthetic materials, such as carboxymethylchitosans and active thrombin preparations; all optionally as fibres bonded to each other. Again, inherently haemostatic absorbent materials used in the device offer convenience in manufacturing the device, since a further therapeutic agent does not have to be incorporated into it.

Alternatively or additionally the device may comprise a therapeutic agent, normally in the absorbent component, for example, an antimicrobial, including antibiotic and/or antimycotic, agent, such as silver, iodine or chlorhexidine.

Alternatively or additionally the device may comprise a haemostatic agent, or an agent that improves scar resolution and/or prevents scar formation, for example: insulin, vitamin B, hyaluronic acid, mitomycin C, growth factors (TGF[beta]), cytokines, corticosteroids and/or agents that promote re-epithelialisation.

The substance can be incorporated in the absorbent component and/or the absorbent material, before or during the manufacturing process of the device. Where a discrete therapeutic agent has to be incorporated into the device, it may be preferred to incorporate it into the absorbent material of the device. Suitable and preferred materials used in the absorbent component of the absorbent device are as so described hereinabove in relation to the absorbent material in general.

This provides convenience in manufacturing a device, since a therapeutic agent may be readily absorbed into the absorbent material from an aqueous medium in which the absorbent material is substantially insoluble. The skilled person will appreciate that, for regulatory reasons, it is greatly preferred that materials used in the absorbent component of the absorbent device and the therapeutic agent should bond to each other so strongly that leaching of the therapeutic agent from the absorbent material by a relevant bodily fluid that has passed through the proximal face of the absorbent component is hindered or prevented.

It is known to use absorbent and cushioning devices for example an cushioning wound dressing to relieve pressure on a body area which is intact tissue around or comprising a wound precursor, as defined herein, to try to hinder or prevent it developing into tissue of compromised integrity, but with limited success. Examples of such wound dressings include Allevyn Gentle ® and Mepilex Border ® wound dressings, normally used for the treatment of a wide range of exuding wounds, such as foot ulcers, pressure ulcers, surgical incisions, and traumatic wounds, such as skin tears, blisters, abrasions and secondary healing wounds. There is thus a need for an absorbent and cushioning device to provide not only treatment of wounds by absorbing bodily fluids, such as exudate, but also to provide relief of pressure on pressure-sensitive areas of intact skin on the body that are particularly subject to pressure, and so provide treatment of wound precursors, as defined herein, and thus prophylaxis of wounds, such as pressure sores.

We have surprisingly found that the above disadvantages may be overcome by an absorbent and cushioning device which comprises a cushioning component is in direct and/or fluidic contact with the distal face of an absorbent component, without any significant transfer in use to the cushioning component of any fluid absorbed into the absorbent material in the absorbent component, so that resilience of any cushioning material is not significantly impaired.

Accordingly, there is provided a conformable absorbent and cushioning device which comprises an absorbent component, and a cushioning component, characterised in that
a) the absorbent component comprises a hydrophilic polymeric absorbent material which has an absorbency in excess of 20 g/g of 0.9 % saline; and by b) a cushioning component which is a separate integer from, but in direct or fluidic contact with, and at least partially overlies the distal face of the absorbent component.

Preferably the absorbency of the absorbent material is 20 to 30 g/g of 0.9 % saline. More preferably the absorbent material is a pharmaceutically acceptable salt of a poly(acrylic acid), of a poly(methacrylic acid) or a poly(acrylic-methacrylic acid) or a blend thereof.

In the absorbent and cushioning device of this eighth embodiment, when used to absorb a bodily fluid, the absorbency of the absorbent material enables large quantities of bodily fluid to be absorbed into the absorbent material in the device of this eighth embodiment. The cushioning component is a separate integer from, but in direct or fluidic contact with the absorbent component. However, there is no transfer of the fluid from the distal face of the absorbent component to the cushioning component.

The resilient elasticity of the material in the cushioning component is thus unimpaired, and it advantageously remains capable of providing adequate relief of pressure on the relevant body area, for example tissue around a wound.

The absorbent and cushioning device of this embodiment may be used to relieve pressure on a body area which is dry intact tissue around or comprising a wound precursor, as defined herein, to try to hinder or prevent it developing into tissue of compromised integrity, such as a chronic wound; that is, to provide treatment of wound precursors, as defined herein, and thus prophylaxis of pressure wounds.

The absorbent and cushioning device surprisingly provide better prophylaxis than known absorbent and cushioning devices, such as the wound dressings Allevyn Gentle ® and Mepilex Border ®, it is believed, because the highly absorbent material in the absorbent component has a significant effect in regulating transpiration under the dressing and into the cushioning component, which is in direct or fluidic contact with the distal face of the absorbent component. The cushioning component is mounted to be in direct or fluidic contact with, and at least partially overlie, the distal face of the absorbent component. The assembly of the absorbent component and the cushioning component may have a number of structures with different components of the dressing of different materials. All of these assemblies, however, comprise the distal face of the absorbent component at least partially covered by the proximal face of the cushioning component.

In a number of forms a wound dressing:
a) the absorbent component may consist essentially of an absorbent material, or b) the absorbent component may comprise the absorbent material, contained within a first container, which is a stand-alone envelope, and
c) the cushioning component may consist essentially of an elastically resilient cushioning material, or
d)
e) the cushioning component may comprise the elastically resilient cushioning material, contained within a second container, which is a stand-alone envelope,

Examples of suitable materials for an absorbent material include synthetic polymers, such as pharmaceutically acceptable salts of poly(acrylic and/or methacrylic) acids. Such materials are favourably cross-linked polyacrylate and polymethacrylate salts, for example alkali metal salts thereof, and copolymers and blends thereof. Preferred absorbent materials include cross-linked polyacrylate and polymethacrylate sodium salts, in particular cross-linked sodium polyacrylates, and blends thereof.

Typically the absorbency of the absorbent material is in excess of 20, such as 20 to 30 g/g of 0.9 % saline. Although this enables large quantities of bodily fluid (g/g of absorbent material in the device) to be absorbed into the absorbent material in the device, the nature and properties of this material prevent transfer of the fluid from the distal face of the absorbent component to the cushioning component.

This in turn, in a non-medical device, such as for the personal care sector, prevents pressure sores forming, or in a medical device, such as a wound dressing, prevents pressure-sensitive chronic wounds, such as diabetic or venous leg ulcers, from worsening. Similarly, when used to relieve pressure on a body area which is dry intact tissue around or comprising a wound precursor, as defined herein, provide treatment of wound precursors, as defined herein, and thus prophylaxis of wounds, such as pressure sores, it is believed that the absorbent and cushioning device may surprisingly provide better prophylaxis than known absorbent and cushioning devices, because the highly absorbent material in the absorbent component has a significant effect in regulating transpiration under the dressing and into the cushioning component.

Suitable and preferred forms of the substantially water-insoluble absorbent material, any conformable container and any conformable backing layer in this eighth embodiment of the device are as so described hereinabove in relation to the present device in general.

The function of the cushioning component in a device is typically to provide relief of pressure on pressure- sensitive areas, rather than to support the area. Accordingly, the conformable absorbent and cushioning device is not often shaped to conform in use to the relevant part of the body.

The device is thus conveniently in a versatile lamellar shape such as an elongate strip, band or ribbon, a quadrilateral, such as a rectangle or oblong, or an equilateral, such as a square. This is referred to herein as an 'unshaped device'. In the unshaped device, the absorbent component and cushioning component are also conveniently in a lamellar shape, referred to herein as an 'unshaped component'. The proximal face of the cushioning component may be smaller than the distal face of the absorbent component. Preferably, however, the faces of the absorbent component and of the cushioning component are essentially coterminous.

In both cases, however, as noted hereinbefore, there should be no appreciable gap between any mutually abutting faces of any of the components of the device, so that the cushioning component will only be in direct or fluidic contact with the absorbent material, and not with the relevant bodily fluid. We have identified conventional cushioning components having an architecture, for example a foam, and being of materials, that make them elastically resilient, but which suffer loss of the initial elastic resilience if a bodily fluid, for example wound fluid, is able to infiltrate into and be retained by the cushioning material. In combination with the absorbent material they suffer no or negligible loss of the initial elastic resilience and also demonstrate good dimensional stability in the present absorbent and cushioning device, for example a cushioning wound dressing.

The cushioning component may comprise a cushioning material which is contained in its own conformable liquid-permeable container, optionally an inner container within an outer container. However, the cushioning component typically consist essentially of, a cushioning material.

As noted hereinbefore, the cushioning component on the distal face of the absorbent component is often an unshaped component in an unshaped device. The cushioning material may be in a range of various forms, and may be in the form of a foam, fibres, an, often non-woven, fabric or a more random assembly of fibres, for example of randomly spun fibres, preferably a foam, in particular an open-cell foam.

Such materials tend to be substantially water-insoluble, elastically resilient synthetic polymeric materials including, but not limited to, hydrophilic polyurethanes, and copolymers and blends thereof. Where the material of the cushioning component comprises a copolymer, examples of suitable copolymers include block copolymers of butylene with styrene. Where the material in the cushioning component is a foam, it may be of the substantially water-insoluble, elastically resilient synthetic polymeric materials described above, including, but not limited to, hydrophilic polyurethane foams, including polyether polyurethane foams, typically the softest of grades with densities from 15 to 30 Kg/m3 , and polyester polyurethane foams, typically with a more controlled cell structure with densities from 20 to 60Kg/m3 ; and polyalkylene foams, including expanded high and low density polyethylene, for example with a small number of long chain branches, and copolymers thereof, for example with a-olefins and functionalised a-olefins, such as vinyl acetate; and copolymers of butylene, including block copolymers of butylene with styrene; and blends thereof.

The cushioning component may additionally or alternatively comprise a microcellular foam material, often a microcellular polyurethane high density foams with good resistance to compression set, often a hydrophilic polyurethane foam, including a polyether polyurethane foam or a polyester polyurethane foam.

The cushioning component may additionally or alternatively comprise a reticulated foam as defined hereinbefore, that is, a foam material which is insoluble in a relevant bodily fluid, and is a soft, flexible, viscoelastic material with a structure with large open cells. It is often a hydrophilic polyurethane foam, including a polyether polyurethane foam, typically of 10 ppi to 60 ppi, or a polyester polyurethane foam, typically of 10 ppi to 90 ppi.

The cushioning component may additionally or alternatively comprise a memory foam as defined hereinbefore, that is, a foam material, which is insoluble in a relevant bodily fluid, and a soft, flexible, resiliently elastic material, often a higher- density viscoelastic low-resilience polyurethane foam with additives which increase its viscosity and density and with an open-cell solid structure that matches pressure against it, and which in contact with a warm mammalian body preferably softens in reaction to body heat, allowing it to mould to the body in a few minutes, yet slowly springs back to its original shape when the pressure is removed.

Such a memory foam may be a gel memory foam, which consists of gel particles fused with memory foam to reduce body heat trapped under the dressing, speed up spring back time and help the dressing feel softer.

The cushioning component may comprise one or more, preferably one, layers of a substantially water-insoluble, elastically resilient polymeric material, optionally bonded to each other. The layer, or optionally bonded layers, may be attached directly to the proximal surface of a backing layer, for example with a pressure sensitive adhesive.

The total layer may typically have a mean pre-absorption thickness of the order of less than 10 mm, for example 4 to 8, such as about 6 millimetres thick.

In a ninth, preferred embodiment, a device of the eighth embodiment comprises a cushioning component which comprises a foam cushioning material, preferably one layer of a foam, in particular an open-cell foam. The total layer may typically have a mean pre-absorption thickness of the order of less than 10 mm, for example 4 to 8, such as about 6 millimetres thick. The mean pre-absorption pore density may be for example from about 2 to 8, particularly from about 3 to 5 pcf. Suitable and preferred materials used in the cushioning component of the absorbent and cushioning device are as so described hereinabove in relation to the cushioning material in general.

In a first form of the absorbent and cushioning device, the foam material used in the cushioning component is a single layer of foam. Suitable and preferred materials are as so described hereinabove in relation to such cushioning material in in that form.

In a second form of the absorbent and cushioning device, the cushioning component comprises a layer of a first foam material and a layer of a second, different foam material. Suitable and preferred materials are as described hereinbefore in relation respectively to such cushioning materials. In this form of this embodiment, the two foam materials are usually present as two layers. The distal layer may be attached directly to the proximal surface of a backing layer, for example with a pressure sensitive adhesive. The two foam layers may be bonded to each other and optionally both be within a conformable (outer) container in the form of a stand-alone envelope, having a proximal cover layer and distal back layer, both permeable to bodily fluid. This may also contain the absorbent component in a conformable stand-alone inner container containing the absorbent material.

Alternatively, the two foam layers may not be bonded to each other. They may both be within a conformable (outer) container in the form of a stand-alone envelope, having a proximal cover layer and distal back layer, both permeable to bodily fluid. This may also contain the absorbent component in a conformable stand-alone inner container containing the absorbent material. Alternatively, when the two foam layers are not bonded to each other, one may be within a conformable (outer) outer container in the form of a stand-alone envelope, having a proximal cover layer and distal back layer, both permeable to bodily fluid. This may also contain the absorbent component in a conformable stand-alone inner container containing the absorbent material. The other foam layer will then lie distally of and outside the (outer) container. The total layer may typically have a mean pre-absorption thickness of the order of less than 10 mm, for example 4 to 8, such as about 6 millimetres thick.

The cushioning component may alternatively comprise a first foam, partly or wholly enclosing a second foam, for example to form a core within the latter within the device. Again the total layer may typically have a mean pre-absorption thickness of the order of less than 10 mm, for example 4 to 8, such as about 6 millimetres thick. In a third form of the absorbent and cushioning device, the foam material used in the cushioning component is an open-cell memory foam as a single layer, and suitable and preferred materials are as so described hereinabove in relation to such cushioning material in in that form.

In a fourth form of the absorbent and cushioning device, the cushioning component comprises
a) an open-cell memory foam as defined hereinbefore, and
b) an open-cell non-memory foam.

Suitable and preferred materials are as described hereinbefore in relation to such cushioning materials. In this form of this embodiment, the two foam materials may be present as two layers, in either order distally. The distal layer may be attached directly to the proximal surface of a backing layer, for example with a pressure sensitive adhesive. The two foam layers may be bonded to each other and optionally both be within a conformable (outer) container in the form of a stand-alone envelope, having a proximal cover layer and distal back layer, both permeable to bodily fluid. This may also contain the absorbent component in a conformable standalone inner container containing the absorbent material.

Alternatively, the two foam layers may not be bonded to each other. They may both be within a conformable (outer) container in the form of a stand-alone envelope, having a proximal cover layer and distal back layer, both permeable to bodily fluid. This may also contain the absorbent component in a conformable stand-alone inner container containing the absorbent material.

Alternatively, when the two foam layers are not bonded to each other, one may be within a conformable (outer) outer container in the form of a stand-alone envelope, having a proximal cover layer and distal back layer, both permeable to bodily fluid. This may also contain the absorbent component in a conformable stand-alone inner container containing the absorbent material. The other foam layer will then lie distally of and outside the (outer) container.

In this form, in the cushioning component, the memory foam will often lie distally of the non-memory foam. The total layer may typically have a mean pre- absorption thickness of the order of less than 10 mm, for example 4 to 8, such as about 6 millimetres thick.

The cushioning component may alternatively comprise a memory foam as defined hereinbefore, partly or wholly enclosed by a non-memory foam, for example to form a core within the latter within the device. Less usually the memory foam may partly or wholly enclose the non-memory foam, for example for the latter to form a core within the former. Again the total layer may typically have a mean pre-absorption thickness of the order of less than 10 mm, for example 4 to 8, such as about 6 millimetres thick. In a fifth form of the absorbent and cushioning device, the cushioning component comprises
a) an open-cell foam, and distally thereof
b) a lamina of conformable, elastic material distally thereof.

Suitable and preferred foam materials are as described hereinbefore in relation respectively to such cushioning materials. The lamina may be of a soft, pliable, almost viscous fluid-like plastics material, preferably of a conformable known gel or elastomer of this type. The gel material, for example, may consist of a silicone-based gel or another appropriate elastomer, for example one based on one or more alkylenes, such as ethylene, propylene and butylene, and styrene and combinations thereof, and thermoplastic polyurethanes, and certain copolyesters and polyamides, and combinations thereof.

Suitable silicone-based gel materials include cross-linked polydimethylsiloxanes which may optionally comprise up to 90 wt%, for example up to 60 wt%, such as up to 40 wt% of an oil, for example a silicone oil, for example a linear polydimethylsiloxanes. These gels have high durability and a useful level of tack, and are characteristically extremely soft and pliable, and can readily be shaped to conform in use to the body. Suitable elastomers include for example one based on a combination of one or more alkylenes, such as ethylene, propylene and butylene, and styrene, in particular styrene ethylene butylene styrene (SBES), and thermoplastic polyurethanes. These are characteristically extremely soft and pliable. Such plastics materials do not generally have good permeability to water vapour. It may thus be desirable to provide a plurality of channels running within the lamina between its proximal face and its opposite distal face and opening in a plurality of pores respectively on the proximal face and its opposing distal face. Mean density values of 1 to 15 per cm2 and mean cross-sectional area values of 0.3 to 10 mm2 may be suitable.

In this form of this embodiment, the distal lamina may be attached directly to the proximal surface of a backing layer, for example with a pressure sensitive adhesive.

The lamina and layer may be bonded to each other and optionally both be within a conformable (outer) container in the form of a stand-alone envelope, having a proximal cover layer permeable to bodily fluid. This may also contain the absorbent component in a conformable stand-alone inner container containing the absorbent material. Alternatively, the lamina and layer may not be bonded to each other. They may both be within a conformable (outer) container in the form of a stand-alone envelope, having a proximal cover layer and distal back layer, both permeable to bodily fluid. This may also contain the absorbent component in a conformable stand-alone inner container containing the absorbent material.

Alternatively, when the lamina and layer are not bonded to each other, the layer may be within a conformable (outer) container in the form of a stand-alone envelope, having a proximal cover layer and distal back layer, both permeable to bodily fluid. This may also contain the absorbent component in a conformable stand-alone inner container containing the absorbent material. The lamina will then lie distally of and outside the (outer) container.

The lamina and layer together may typically have a mean pre-absorption thickness of the order of less than 10 mm, for example 4 to 8, such as about 6 millimetres thick.

In a sixth form of the absorbent and cushioning device, the cushioning component consists essentially of a foam material (including a memory foam material) as described hereinbefore. Suitable and preferred materials are as so described hereinabove in relation to such cushioning material in that form.

In all forms of this embodiment, preferably
a) a conformable outer container in the form of a stand-alone envelope, having a proximal cover layer and distal back layer, both permeable to bodily fluid, contains the assembly of
   i) the absorbent component in a conformable inner container containing the absorbent material in the form of a stand-alone envelope, having a proximal cover layer and distal back layer, both permeable to bodily fluid, and
   ii) the cushioning component,
b) the back layer of the outer container lies in direct contact with and is attached directly on its distal face to the proximal face of
c) a liquid-impermeable device backing layer with a high moisture vapour transmission (MVT), of at least 450g/m2 /24hr. Optionally a body contact layer in the form of a perforated layer overlies and extends beyond the proximal face of the absorbent component and is attached directly to the periphery of the proximal face of the backing layer.

The present invention provides a method of manufacturing a conformable absorbent wound dressing, which comprises a heat-plasticising process, which comprises subjecting the proximal face of a wound contact layer of a precursor of a device to heat, and pressure, sufficient to soften it.

A variant of the heat-plasticising process comprises subjecting the proximal face of a wound contact layer to heat, and pressure, sufficient to soften it, and then attaching it to another precursor of a device, often directly or indirectly to a liquid-impermeable backing layer of the device precursor.

In this variant of the heat-plasticising process, the proximal face of the wound contact layer in a precursor may be subjected to pressure, since no wound contact integer is present, and thus no the risk of compromising the performance of the components and materials thereof.

The wound contact layer in a precursor of a device is liquid-permeable, and typically comprises at least one, preferably one, perforated layer or sheet, or layer of spun-bonded or non-woven fabric, of a flexible and relatively inelastic polymer material.

Where the layer is a layer of spun-bonded or non-woven fabric, it will often have a monofilament linear density of 0.1 to 30, preferably about 0.5 to 20, and more preferably 0.9 to 4, for example 1 3 to 5 decitex. It will have a strength of 0.8 to 2.2, such as 1 to 2, for example 1 .2 to 1 .8 cN/dtex.

The material of the wound contact layer, whether as a perforated layer or sheet, or layer of spun-bonded or non-woven fabric, is typically a flexible and relatively inelastic material.

Examples of suitable materials include substantially water-insoluble synthetic thermoplastic polymeric materials, such as polyalkylenes, for example polyethylenes, polypropylenes and polybutylenes, polyoxalates, polyamides, silicones, polyurethanes, polyesters and copolymers and blends thereof. Both variants of the heat-plasticising process may best be carried out by placing the proximal face of the wound contact layer on a heated platen, and subjecting the proximal face to heat sufficient to soften it,.

The parameters in the heat-plasticising process within the method of manufacturing an absorbent device include
a) a platen temperature of 120 to 180°C, such as 130 to 170°C, for example 140 to 160°C,
b) a pressure of 3 to 9 Bar, such as 4 to 8 Bar, such as 5 to 7 Bar, and
c) a dwell time of the wound contact layer in the process of 2 to 9 seconds, such as 3 to 8 seconds, for example 4 to 7 seconds, depending on the structure and materials of the wound contact layer.

In the variant of the heat-plasticising process, the step of attaching the heat-plasticised wound contact layer to the liquid-impermeable backing layer of the device precursor may be carried out as described hereinabove in relation to these components in general, for example with a pressure sensitive adhesive,

The dressing may be used in a method on a body area from where bodily fluids are to be absorbed, characterised by the application of a dressing to the body area.

The dressing can be easily applied to treat a precursor of a wound, and thus to provide prophylaxis of a wound, for example a pressure sore, an ulcer, such as a diabetic foot ulcer or venous leg ulcer, or a burn wound.

The present invention is illustrated by the following Figure, which shows a cross sectional side view of one form of a device manufactured by the invention.

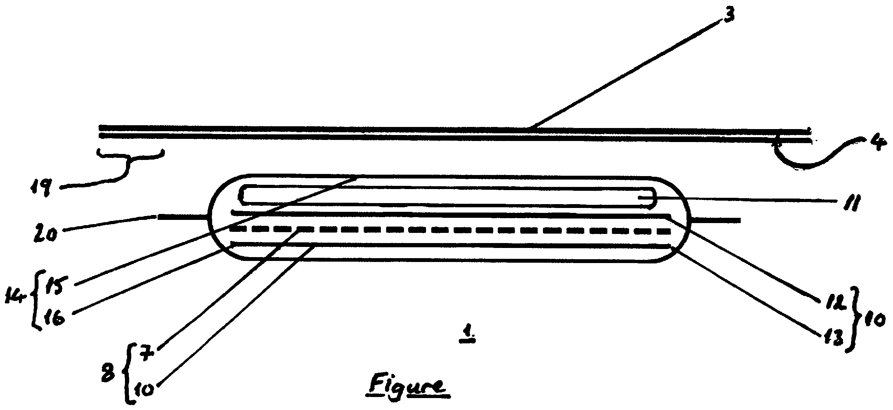

Referring to the Figure, a conformable absorbent and cushioning device, here a dressing 1, comprises an absorbent component 8 and a cushioning component 11, in which
a) the absorbent component 8 comprises a hydrophilic polymeric absorbent material 7 which is a pharmaceutically acceptable salt of a poly(acrylic acid), a poly(methacrylic acid) or a poly(acrylic-methacrylic acid) or a blend thereof, and
b) a cushioning component 1 1 , which is a separate integer from, but in fluidic contact with, and at least partially overlies the distal face of, the absorbent component 7.

The absorbent component 8 comprises an absorbent material 7, here a particulate of a substantially water-insoluble hydrophilic material, here a sodium polyacrylate superabsorbent. The material 7 is contained in an inner container 10 in the form of a stand-alone envelope.

The distal 12 and proximal 13 faces of the container 10 (respectively the back layer and the cover layer) are of a water-insoluble, non-woven fabric, here of a blend of spun-bonded flexible fibres of a polyethylene - polyethylene terephthalate.

The cushioning component 1 1 consists essentially of a layer of an open-cell foam, here a soft-grade hydrophilic polyether polyurethane foam (alternatively a hydrophilic polyester polyurethane foam). The absorbent component 8 comprising the absorbent material 7 in the inner container 10 and the cushioning component 1 1 are in turn contained in an outer container 14, also in the form of a stand-alone envelope, together forming a wound contact integer. The distal face 15 of the container 14 (the back layer) is made of the same material as that of the inner container 10.

The proximal face 16 of the container 14 is a fluid-permeable wound contact layer. It is made of the same material as that of the inner container 10, but its proximal face comprises a heat-plasticised form of the material.

The material in the face 16 has been subjected to heat and pressure, sufficient to soften it, by subjecting the proximal face 16 of the wound contact layer in a precursor of the wound contact integer comprised in the stand-alone envelope 14. This is described in the Example hereinafter.

The dressing 1 also comprises an elastically resilient gas-permeable, liquid-impermeable, relatively high-MVT film backing layer 3, here a polyurethane film layer.

The backing layer 3 overlies and extends beyond the periphery of the outer container 14 to form a border 19 on a part of the proximal face 4 of the backing layer 3, around the periphery 20 of the outer container 14. After processing, the distal face of the back layer 15 of the container 14 has been attached directly to the proximal face of the device backing layer 3. A layer 17 of pressure-sensitive adhesive, here a pressure sensitive acrylic adhesive, extends across the proximal face 4 of the backing layer 3 and across the border 19, and is the means by which the proximal face 4 of the backing layer 3 is directly adhered to the absorbent component 6.

On the border 19 around the periphery 20 of the outer container 14, it is used for the purpose of securing the backing layer 3 of the dressing 1 over a wound, to form a relatively liquid-tight closure to exudate from the wound. It is covered with release papers (not shown) release-coated with a silicone, when the dressing 1 is not in use.

In use, the silicone-coated release papers are removed from the layer 17 of pressure-sensitive adhesive on the border 19 around the periphery 20 of the outer container 14, and the pressure sensitive acrylic adhesive is used to secure the conformable device backing layer 3 of the dressing 1 to the body of a patient across a wound, for example a pressure sore, or an ulcer, such as a diabetic foot ulcer. Exudate absorption through respectively the proximal 16, 13 faces of the outer container 14 and of the inner container 10 and its absorption into and retention by the absorbent material 7 in the inner container 10 occurs.

Although the cushioning component foam 1 1 is in direct contact with the absorbent component 8, when the latter is saturated the foam 1 1 remains dry, and the resilient elasticity of the material in the cushioning component 1 1 is unimpaired on exudate absorption and retention by the dressing. The foam therefore remains able to act as a cushion and dissipate pressure, giving constant pressure relief through wearing the dressing.

The present invention is also illustrated by the following Example:

### Example

The heat-plasticising process within the method of manufacturing an absorbent device was carried out as follows to form the wound contact integer of a device.

A heat sealing / welding machine was used with a square-section well-shaped jig in a lower platen with an upper sealing plate cooperating with the area surrounding the rim of the well, and in which the area surrounding the rim of the well, and the sealing plate can be heated, and in which pressure may be applied by the sealing plate to the area surrounding the well. The following components of the wound contact integer of a device were laid in order from the bottom up over the well of the jig:
A back layer of the outer stand-alone envelope, consisting of a layer of a blend of spun-bonded flexible fibres of a polyethylene - polyethylene terephthalate, which extends beyond the periphery of the well to form a border around the periphery of the well.

A cushioning component consisting of a layer of an open-cell soft-grade hydrophilic polyether polyurethane foam, with a periphery that is co-extensive with the periphery of the well

An absorbent component comprising an absorbent material particulate of a substantially water-insoluble hydrophilic sodium polyacrylate superabsorbent, contained in an inner stand-alone envelope, the back layer and the cover layer of which are of a blend of spun-bonded flexible fibres of a polyethylene - polyethylene terephthalate, and with a periphery that is co-extensive with the periphery of the well and the cushioning component.

A precursor of a wound contact layer and cover layer of an outer stand-alone envelope, consisting of a layer of a blend of spun-bonded flexible fibres of a polyethylene - polyethylene terephthalate.

This extends beyond the periphery of the well to form a border around the periphery of the well and is co-extensive with the periphery of the back layer

The heated components of the machine were pre-heated for 30 min. to 150° C. A pressure of 6 Bar with a dwell time of 5.5 seconds was then applied between the pusher pin and the area surrounding the rim of the well to heat-seal the periphery of the back layer to the periphery of the cover layer. The face of the cover layer is plasticised by the heated surface of the sealing plate.

## Claims

1. A method of manufacturing a conformable absorbent wound dressing, said dressing comprising:
a wound contact integer comprising an absorbent component and optionally a cushioning component, the wound contact integer having a proximal face and a distal face;
a liquid-impermeable backing layer which overlies the distal face of the wound contact integer, and;
a liquid-permeable wound contact layer which overlies the proximal face of the wound contact integer, wherein the proximal face of the wound contact layer comprises a heat-plasticised, resiliently elastic material;
**characterised in that** the method comprises a heat-plasticising process, which comprises subjecting the proximal face of a wound contact layer of a precursor of the wound dressing or a wound contact layer of a precursor of the wound contact integer, to heat, and pressure, sufficient to soften it by placing the proximal face of the wound contact layer on a heated platen with a platen temperature of 120 to 180°C, a pressure of 3 to 9 Bar, and a dwell time of the wound contact layer on the platen of 2 to 9 seconds.

## Patentansprüche

1. Verfahren zum Herstellen eines anpassungsfähigen absorbierenden Wundverbands, wobei der Verband Folgendes umfasst:
eine Wundkontaktauflage, die eine absorbierende Komponente und optional eine Polsterungskomponente umfasst, wobei die Wundkontaktauflage eine proximale Fläche und eine distale Fläche aufweist;
eine flüssigkeitsundurchlässige Rückschicht, die über der distalen Fläche der Wundkontaktauflage liegt, und;
eine flüssigkeitsdurchlässige Wundkontaktschicht, die über der proximalen Fläche der Wundkontaktauflage liegt, wobei die proximale Fläche der Wundkontaktschicht ein wärmeplastifiziertes, nachgiebiges, elastisches Material umfasst;
**dadurch gekennzeichnet, dass** das Verfahren einen Wärmeplastifizierungsprozess umfasst, der umfasst, die proximale Fläche einer Wundkontaktschicht eines Vorprodukts des Wundverbands oder eine Wundkontaktschicht eines Vorprodukts der Wundkontaktauflage Wärme und Druck auszusetzen, die ausreichend sind sie zu erweichen, indem die proximale Fläche der Wundkontaktschicht auf einer erwärmten Platte mit einer Plattentemperatur von 120 bis 180°C bei einem Druck von 3 bis 9 Bar und für eine Verweilzeit der Wundkontaktschicht auf der Platte von 2 bis 9 Sekunden platziert wird.

## Revendications

1. Procédé de fabrication d'un pansement pour plaie absorbant adaptable, ledit pansement comprenant :
une partie intégrante en contact avec la plaie comprenant un composant absorbant et éventuellement un composant de garnissage, la partie intégrante en contact avec la plaie ayant une face proximale et une face distale ;
une couche de fond imperméable aux liquides qui recouvre la face distale de la partie intégrante en contact avec la plaie, et ;
une couche de contact avec la plaie perméable aux liquides qui recouvre la face proximale de la partie intégrante en contact avec la plaie, dans lequel la face proximale de la couche en contact avec la plaie comprend un matériau élastique thermoplastifié ;
**caractérisé en ce que** le procédé comprend un processus de thermoplastification, qui comprend la soumission de la face proximale d'une couche de contact avec la plaie d'un précurseur du pansement pour plaie ou d'une couche de contact de plaie d'un précurseur de la partie intégrante en contact avec la plaie, à un chauffage, et une pression, suffisants pour l'assouplir en plaçant la face proximale de la couche de contact avec la plaie sur une plaque chauffée avec une température de plaque de 120 à 180 °C, une pression de 3 à 9 bars, et un temps de séjour de la couche en contact avec la plaie de 2 à 9 secondes.
